# EUROPEAN PATENT APPLICATION

(11) **EP 3 354 286 A1**
(43) Date of publication of application: **01.08.2018**
(21) Application number: 17153699.8
(22) Date of filing: 30.01.2017
(51) Int. Cl.: A61L 2/00, A61L 2/18, A61L 2/20, B08B 9/032, A61L 11/00

(54) **NON-STERILE WASTE REMOVAL FROM A STERILE PROCESS**

(71) Applicant: Bayer Healthcare LLC, Whippany, NJ 07981 (US)
(72) Inventor: Schwan, Peter, 51373 Leverkusen (DE); Vogt, Andreas, 50969 Köln (DE); Weber, Andreas, 50733 Köln (DE); Sievers, Stefan, 40627 Düsseldorf (DE); Lobedann, Martin, 51069 Köln (DE)
(74) Representative: BIP Patents

(57) **Abstract**

Disclosed herein is a device comprising at least one pathogen-reduced collection container (1), which is in fluid connection with at least one non-sterile waste disposal site (16) via at least one distributor comprising at least three attachment points for tubes (2,3) and a method for using the this device.

## Description

Various processes such as production processes e.g. for medical products require closed, pathogen-reduced or even sterile conditions. Components and materials used in such processes, but which are no longer required, are usually led into a waste container e.g. a sterile bag, which is in pathogen-reduced or even sterile connection with the process. Once the bag is full, it has to be removed i.e. separated from the process under pathogen-reduced or even sterile conditions and a new waste container has to be connected while maintaining pathogen-reduced or even sterile conditions.

However, this process is time-consuming, tedious, potentially prone to error if handled by unexperienced users and expensive, especially since all items have to be sterilized.

Alternatively, if the production process results in a waste stream this waste stream could in principle be sanitized and/or disinfected using methods such as electrochemical oxidation, low pressure oxidation (LOPROX) or ozonization. In order to maintain sterility the process has to be closed and all of the mentioned methods carry the risk of gas and/or foam formation. This is disadvantageous as removing gas and foam from a closed process may be difficult and time-consuming.

Moreover, methods for irradiating water from aquariums with UV-C in order to ensure a microbe-reduced environment are known in the art.

In addition, flow-through autoclaving devices are available, which use temperatures of 121 °C for sterilization.

Alternatively, a waste stream may be removed from a process via several sterile filters mounted in parallel, alternating, in series or in a combination of these settings. However, such an approach is expensive due to high material and maintenance cost for sterile filters as well as the fact that with precipitations the filters are easily blocked.

Therefore, there is a need for a simplified system that allows a faster, more efficient and cost reduced waste removal from a pathogen-reduced or even sterile process.

For the first time it was surprisingly found that these objectives can be met by a system, in which the input side of the waste disposal site is pathogen-reduced and/or sterile but the waste disposal site itself and its exit does not have to be sterile and/or pathogen-reduced.

In one aspect the present inventions relates to a device comprising at least one pathogen-reduced collection container, which is in fluid connection with at least one non-sterile waste disposal site via
- at least one distributor comprising at least three attachment points for tubes,
- at least three tubes, one of which connects the collection container to the first of said at least three attachment points for tubes of the at least one distributor,
- the second tube connects the second of said at least three attachment points for tubes of the at least one distributor with the non-sterile waste disposal site, and
- the third of said three tubes connects the third of said at least three attachment points for tubes of the at least one distributor with at least one sanitization source, comprising sanitization medium to decontaminate at least part of the second tube.

Under non-sterile conditions i.e. without the requirement that the collection container remains pathogen-reduced the direct fluid connection between the collection container and the waste disposal site could be a simple tube. However, as the conditions in the collection container need to be pathogen-reduced and if possible sterile in order to ensure the pathogen-reduced state of the production process connected (upstream) to the collection container, the risk of contamination entering the collection container or the tubes leading to the collection container via the non-sterile waste disposal site has to be eliminated or at least minimized.

It was surprisingly found, that said risk can be minimized via using a distributor comprising at least three attachment points for tubes, which is connected to the collection container, at least one non-sterile waste disposal site and a sanitization source in combination with the sanitization medium comprised in said sanitization source. This distributor allows for a fluid connection to the at least one non-sterile waste disposal site thus allowing a faster, more efficient and cost reduced waste removal from a sterile and/or pathogen-reduced process. At the same time the risk of contamination entering the collection container or the tubes leading to the collection container via the at least one non-sterile waste disposal site is minimized and/or eliminated due to sanitization with the sanitization medium comprised in the at least one sanitization source, which is used to decontaminate at least part of the second and third tubes whenever required, e.g. in regular time intervals or when a threshold of contamination is reached.

As used herein, the expression "at least one" means one or more.

It is also to be understood that, as used herein the terms "the," "a," or "an," mean "at least one," are understood to encompass the plural as well as the singular and should not be limited to "only one" unless explicitly indicated to the contrary.

As used herein the term "distributor comprising at least three attachment points for tubes" refers to a device, which enables control of the fluid connection between the collection container and the at least one non-sterile waste disposal site. In other words, via regulating the distributor comprising at least three attachment points for tubes the flow of a fluid stream from the collection container to the at least one non-sterile waste disposal site can be controlled and decontamination is allowed e.g. at a given time point i) a fluid stream flows from the collection container to the at least one non-sterile waste disposal site. At a given time point ii) the fluid stream is stopped at and/or in the distributor comprising at least three attachment points for tubes and sanitization medium flows from the sanitization source to the at least one non-sterile waste disposal site via the distributor comprising at least three attachment points for tubes, thereby decontaminating at least the second and third tubes.

In addition, the distributor comprising at least three attachment points for tubes enables distribution of the fluid stream. This is for example achieved via a T-piece and/or an Y-piece and/or a three-way valve and/or multiple-way-valve comprised in the distributor comprising at least three attachment points for tubes. In other words in the case of a T-piece, for instance, the three arms of said T-piece represent the three attachment point for tubes of the distributor.

As used herein the term "fluid connection" refers to the fact that the fluid contained in the collection container of the device described herein can pass into the at least one non-sterile waste disposal without being collected again.

As used herein the term "tube" is used interchangeably with the term "tubing" or "line of tubing".

As used herein the term "fluid stream" or "fluid flow" refers to a flow of liquid and/or gas, which may contain solids like salts, flocculations, precipitations or crystals. Thus the waste stream flowing from the at least one collection container to the at least one non-sterile waste disposal site via the at least one distributor comprising at least three attachment points for tubes is an example of a fluid stream.

As used herein the term "non-sterile waste disposal" refers to a component, which is not sterile and/or pathogen-reduced, into which the waste of the collection container is transferred and in which said waste is disposed.

An example of such a non-sterile waste disposal is a drain. Said drain can comprise a funnel as inlet.

As used herein the term "collection container" refers to a container, in which the waste resulting from one or more unit operations to which the device described herein can be connected can be collected.

In a preferred embodiment said collection container is selected from the group comprising a sterilizable plastic bag, a plastic container, a sterilizable 3-D bag or a closed stainless steel vessel.

Preferably the collection container is a form-stable, sterilizable and disposable plastic bag.

For example said collection container can hold between 1-1000 liters, between 10-300 liters and most preferably 20-200 liters.

For example the collection container is a gamma-irradiated 200 liter Sartorius Flexel.

The type of collection container and the rate with which it is filled determine the rate with which the fluid has to flow from the collection container via the distributor comprising at least three attachment points to the waste disposal site, since an overflow of the collection container should be avoided.

As used herein the term "pathogen-reduced" is used interchangeable with "microbe-reduced" and "germ-reduced" and refers to a state of reduced pathogenic count, i.e. a pathogenic count per area or volume unit of close to zero that is achievable by means of a suitable germ-reducing method, wherein this germ-reducing method can be selected from gamma irradiation, beta irradiation, autoclaving, Ethylene Oxide (ETO) treatment, Ozone treatment, "Steam-In-Place" (SIP) and/or Heat in Place treatment or treatment with sanitization agent like 1 M NaOH.

As used herein the term "sanitization source" is a tank and/or reservoir holding a sanitization medium in liquid or gaseous form.

One example of a sanitization medium is 1 M NaOH solution.

As used herein the term "sanitization" or "decontamination" refers to the process of achieved a sterile and/or pathogen-reduced state. This does not necessarily involve cleaning of a given material.

Instead decontamination can be achieved via inactivating or destroying a pathogen or a contamination ("disinfecting"). However, decontamination an also refer to both inactivating or destroying a pathogen or a contaminantion and also removing said pathogen or contaminantion, i.e. cleaning of the material.

In general, the type of sanitization medium and the characteristics of the second tube determine the prerequisites for decontamination. This is the case as due to the choice of sanitization medium the time required for reliably decontaminating especially the second tube can vary. For example decontamination can be achieved via flushing the second and third tube with 10x their hold-up volume of sanitization medium.

Usually at least part of the second tube has to be decontaminated with sanitization medium in order to eliminate or minimize the risk of contamination entering the collection container or the tubes leading to the collection container via the at least one non-sterile waste disposal site. However, depending on factors such as the type of fluid flowing in the tubes, the type of at least one non-sterile waste disposal site, the length of the time interval between decontaminations as well as the length of tubing between the collection container and the at least one non-sterile waste disposal site also decontamination of other parts of the device can be necessary and/or advantageous. Since the collection container in general comprises fluid which is to be disposed it should usually be possible to also decontaminate the first tube - via arranging the setting of the distributor comprising at least three attachment points for tubes accordingly - without interfering with unit operation upstream of the device described herein.

As used herein the term "unit" or "unit operation" refers to a device that performs one process step in a production process e.g. in the production process of a biopharmaceutical and biological macromolecular product and to the process which that specific device performs.

The device described herein does not necessarily require one or more pumps to enable fluid flow from the collection container to the waste disposal site. Instead fluid flow may be achieved via gravity e.g. by locating the collection container and the sanitization source above the waste disposal site. In such a setting the hydrostatic pressure enables fluid flow from the collection container into the waste disposal site via the distributor comprising at least three attachment points for tubes.

In a preferred embodiment of the device described herein in which the device does not require pumps to enable fluid flow from the collection container to the waste disposal site the device comprises flow sensors and/or level sensors, e.g. scales, which enable process control and ensure that the collection container does not overflow.

In one embodiment the device described herein comprising at least one pump that pumps the fluid of the collection container to the at least one non-sterile waste disposal site and sanitization medium through the third as well the second tube, respectively.

In one example of this embodiment of the device one pump is located at the second tubing near the waste disposal site. Thus, in a first setting of the distributor comprising at least three attachment points this pump can draw fluid from the collection container via the first tube and the second tube into the waste disposal site. Subsequently, during a second setting of the distributor comprising at least three attachment points, the first tube is not in direct connection with the waste disposal site, instead the third tube is in direct connection with the waste disposal site and hence the pump draws sanitization medium from the sanitization source into the waste disposal site via the third and second tubes.

In one embodiment the device described herein comprises at least two pumps.

In one example of this embodiment the device comprises two pumps, wherein the second pump is used to mix the fluid in the collection container, thereby minimizing the risk that particles form which can potentially block parts of the device such as the tubes leading to the at least one non-sterile waste disposal site.

Moreover, the device can comprise more than two pumps, e.g. three or four pumps.

In one embodiment the at least one distributor comprising at least three attachment points for tubes comprises a T-piece and/or an Y-piece and/or a three-way valve for distribution of the fluid stream.

In one embodiment the at least one distributor comprising at least three attachment points for tubes further comprises a component selected from the group comprising: at least two valves, a combined dual valve and at least one pump, a T-piece, a Y-Piece, a multiple-way-valve, or at least two pumps acting as valves.

It should be noted that is it possible to combine the different types of the at least one distributor comprising at least three attachment points for tubes in order to control the flow of a fluid stream from the collection container to the non-sterile waste disposal site and allow decontamination.

In case that the distributor comprising at least three attachment points for tubes is a T-piece or a Y piece it is preferred that the distributor comprising at least three attachment points for tubes further comprises at least one three-way valve, at least one combined dual valve and at least one pump, or , at least two valves or at least two pumps in order to control fluid flow.

Since in a combined dual valve one flow path is always opened while the other one is closed the pump ensures that fluid flow through all three arms of the T-piece or the Y-piece is controlled.

Hence, in case that the distributor comprising at least three attachment points for tubes is realized as T-piece the device can e.g. be constructed as follows: the first tube connects the collection container with a first arm of said T-piece, the second tube connects the second arm of the T-piece with the non-sterile waste disposal site, and the third of said three pieces of tubing connects third arm of the T-piece with at least one sanitization source, comprising sanitization medium to decontaminate at least part of the second tube if required. During operation a fluid stream can flow from the collection container via the first tube, the T-piece and the second tube into the non-sterile waste disposal site. Likewise sanitization medium can flow from the sanitization source via the third tube and the second tube to the non-sterile waste disposal site, thereby decontaminating the second tube. As mentioned above, it is preferred that in this setting the device further comprises at least one three-way valve, a combined dual valve and at least one pump, or at least two valves, or at least two pumps in order to control if or how far the sanitization medium also flows into the first tube and how far the fluid from the collection container flows into the third tube. However, as the collection container usually contains waste fluid, generally the device can also be operated if sanitization medium flows into the collection container.

Furthermore, in case that the distributor comprising at least three attachment points for tubes is realized as T-piece and comprises at least two valves the device can e.g. be constructed as follows: the first tube connects the collection container via the first of said at least two valves with the first arm of said T-piece, the second tube connects the second arm of said T-piece with the non-sterile waste disposal site e. and the third of said three pieces of tubing connects the non-sterile waste disposal site via the second valve with at least one sanitization source, comprising sanitization medium to decontaminate at least part of the second tube whenever required. During operation a fluid stream can flow from the collection container via the first tube to the first of said at least two valves. If this first valve is open and the second valve is closed, the fluid stream can flow further into the non-sterile waste disposal site via the second tube. If the first valve is closed and the second valve is open the fluid stream is haltered and the sanitization medium can flow from the sanitization source via the third tube and the second valve to the non-sterile waste disposal site, thereby decontaminating the second tube.

Moreover, in case that the distributor comprising at least three attachment points for tubes is realized as T-piece and comprises a three-way valve the device can e.g. be constructed as follows: the first tube connects the collection container with the distributor comprising at least three attachment points for tubes via a first valve of said three-way valve, the second tube connects the distributor comprising at least three attachment points for tubes via a second valve of said three-way valve with the non-sterile waste disposal site, and the third of said three pieces of tubing connects the distributor comprising at least three attachment points for tubes via a third valve of said three-way valve with at least one sanitization source, comprising sanitization medium to decontaminate at least part of the second tube whenever required. During operation a fluid stream can flow from the collection container via the first tube to the first valve of said three-way valve. If this first and the second valves are open and the third valve of said three-way valve is closed, the fluid stream can flow further into the non-sterile waste disposal site via the second tube. If the first valve is closed and the second and third valves are open the fluid stream is haltered and the sanitization medium can flow from the sanitization source to the non-sterile waste disposal site, thereby decontaminating the second tube.

In addition, in case that the distributor comprising at least three attachment points for tubes is realized as T-piece and further comprises least two pumps acting as valves the set-up is identical to the one described above for the at least two valves except that in order to halt fluid flow in a given tube the pump has to pause thereby closing off the tubing.

In one embodiment of the device described herein an air gap is present between the at least one non-sterile waste disposal site and the second tube, which connects the second of said at least three attachment points for tubes of the at least one distributor with the non-sterile waste disposal site.

As used herein the term "air gap" refers to a security measure employed to ensure that a connection leading to a non-sterile waste disposal site is physically apart from said non-sterile waste disposal site. In other words, said air-gap ensures that no contamination present on the surface of the non-sterile waste disposal site can enter the connection, e.g. the tubing - leading to the non-sterile waste disposal site, instead - at least in theory - only air-born contaminations can enter said connection leading to the non-sterile waste disposal site.

A device as described above, wherein the device is a unit operation.

As used herein the term "unit" or "unit operation" refers to a device that performs one process step in a production process of a biopharmaceutical and biological macromolecular product and to the process which that specific device performs. In other words, in order to provide the final biopharmaceutical and/or biological macromolecular product several units will have to be passed by the fluid stream until the product has the desired characteristics and/or purity.

As used herein the term "modular" means that the individual unit operations can be carried out in separate interconnected modules, wherein the modules are preconfigured, germ-reduced, and closed, and can be interconnected in various combinations.

As used herein the term "flow path" refers to any assembly or containment through which the fluid flow passes or is in contact with. For example, the first second and third tubes represent a flow path.

Preferably, times with a low or zero flow in the second tube containing waste fluid of the production process upstream of the device described herein are minimized or avoided. For example said low or zero flow is not allowed to exceed 2 h. However, the situation may occur that all second tubes contain sanitization fluid, i.e. no fluid flows from the collection container towards the non-sterile waste disposal site.

In a preferred embodiment the device comprises more than one distributor comprising at least three attachment points for tubes.

In one example of this embodiment the device has two distributors comprising at least three attachment points for tubes characterized in that said distributors comprise two valves each.

In one example of this embodiment the device has two distributors comprising at least three attachment points for tubes. In one example of this embodiment, the two distributors comprising at least three attachment points for tubes share a sanitization source. Hence the device comprises only one third tubing, but two tubes acting as second tubes, i.e. one tube connecting each of the two distributors with the non-sterile waste disposal site. However, it is also possible, that each distributor comprising at least three attachment points for tubes has a separate sanitization source.

In one example of this embodiment one of the two second tubes contains sanitization medium and the other one contains fluid flow towards the non-sterile waste disposal site or vice versa. This specific example of this embodiment has the additional advantage that it allows uninterrupted flow from the collection container to the non-sterile waste disposal site, since at a given time point a fluid stream can flow directly from the collection container to the non-sterile waste disposal site via the first distributor comprising at least three attachment points for tubes, while the second and third tubes of a second distributor comprising at least three attachment points for tubes are decontaminated. Uninterrupted flow is then achieved via altering the flow path of the fluid stream so that the fluid stream can flow directly from the collection container to the non-sterile waste disposal site via the second distributor comprising at least three attachment points for tubes, while the second and third tubes of a first distributor comprising at least three attachment points for tubes are decontaminated.

This switching of flow paths can for example take place, when decontamination is completed or in regular time intervals.

In one example of such a setting the device comprises two distributors comprising at least three attachment points for tubes and three pumps in total.

As used herein the term "uninterrupted" refers to the fact, that at least one flow path is available so that the fluid stream can steadily enter the at least one non-sterile waste disposal site, without interruption.

In a preferred embodiment all components coming into contact with the fluid flow are disposable articles or are used as disposable articles.

As used herein the term "disposable articles" means that the respective components coming into contact with the fluid stream, particularly equipment, containers, filters, and connecting elements, are suitable for one-time use followed by disposal, wherein these containers can be made of both plastic and metal. Within the scope of the present invention, the term also comprises disposable articles such as those made of steel that are only used once in the process according to the invention and not used again in the process. These disposable articles, for example those made of steel, are then also designated within the scope of the invention as objects "used as disposable articles." Such used disposable articles can then also be designated in the process according to the invention as "disposable" or "single-use" articles ("SU technology"). In this way, the pathogen-reduced status of the process and modular system according to the invention is improved even more.

In one embodiment the device described herein comprises the non-sterile waste disposal site.

Furthermore what is described herein also relates to a production plant comprising one or more devices as described above.

Preferably the production plant is a modular system (1) for the continuous, microbe-reduced production and/or processing of a biopharmaceutical, biological macromolecular product characterized in that the modular system (1) is closed and microbe-reduced.

As used herein the term "continuous" refers to a method for carrying out at least two method steps and/or unit operations in series in which the outlet fluid stream (fluid flow) of an upstream step is transported to a downstream step. The downstream step begins processing the fluid flow before the upstream step is completed. Accordingly, continuous transport or transfer of a fluid flow from an upstream unit to a downstream unit means that the downstream unit is already in operation before the upstream is shut down, i.e. that two units connected in series simultaneously process the fluid flow that is flowing through them

As used herein the term "closed" refers to both "functionally closed" as well as "completely closed".

As used herein the term "completely closed" means that the production plant is operated in such a way that the fluid stream is not exposed to the room environment. Materials, objects, buffers, and the like can be added from outside, wherein, however, this addition takes place in such a way that exposure of the fluid stream to the room environment is avoided.

The term "functionally closed" refers to a process that may be opened but is "rendered closed" by a cleaning, sanitization and/or sterilization that is appropriate or consistent with the process requirements, whether sterile, aseptic or low bioburden. These systems shall remain closed during production within the system. Examples include process vessels that may be CIP'd and SIP'd between uses. Non-sterile systems such as chromatography or some filtration systems may also be rendered closed in low bioburden operations if appropriate measures are taken during the particular system setup.

In one embodiment the biopharmaceutical, biological macromolecular product comprises at least one component selected from the group consisting of a peptide, a protein, a small molecule drug, a nucleic acid.

As used herein the term "peptide" refers to a polymer of amino acids of relatively short length (e.g. less than 50 amino acids). The polymer may be linear or branched, it may comprise modified amino acids, and it may be interrupted by non-amino acids. The term also encompasses an amino acid polymer that has been modified; for example, by disulfide bond formation, glycosylation, lipidation, acetylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

As used herein the term "protein" refers to a polypeptide of amino acids. The term encompasses proteins that may be full-length, wild-type, or fragments thereof. The protein may be human, non-human, and an artificial or chemical mimetic of a corresponding naturally occurring amino acid, as well as to naturally occurring amino acid polymers and non-naturally occurring amino acid polymer. The term also encompasses a protein that has been modified; for example, by disulfide bond formation or cracking, glycosylation, lipidation, acetylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

Preferably the protein is a therapeutic protein.

As used herein the term "therapeutic protein" refers to a protein that can be administered to an organism to elicit a biological or medical response of a tissue, an organ or a system of said organism.

Even more preferably the protein is an antibody.

Moreover, what is described herein also relates to a method for non-sterile waste removal from a microbe-reduced or sterile process using the device described above.

In a one embodiment of this aspect the method for non-sterile waste removal from a microbe-reduced or sterile process comprises the step that prior to initiating non-sterile waste removal from a microbe-reduced or sterile process the device is flushed with sanitization medium.

Such a step is advantageous, since it prevents contamination of the flow paths of the device prior to use.

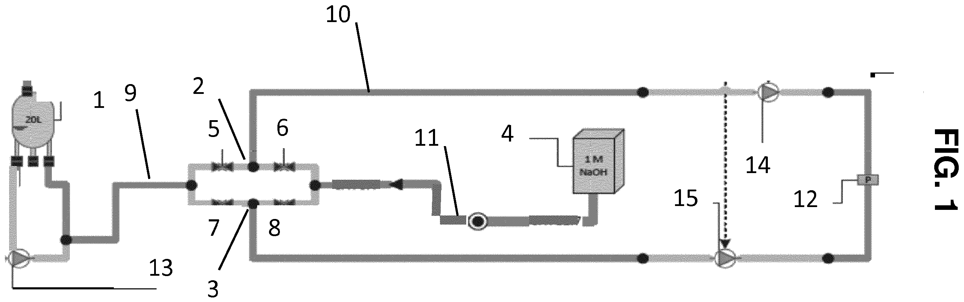

In a preferred embodiment - depicted in FIG. 1 - the device comprises prior to use in a method for non-sterile waste removal from a microbe reduced or sterile process a collection container (1), two distributors comprising each at least three attachment points for tubes - here two T-pieces (2,3) - as well as two sets of valves, i.e. the first distributor comprises two valves (5,6) and the second distributor also comprises two valves (7,8). A first tube (9) connects the first arm of each of the two T-pieces (2,3) via valves (5) and (7) respectively with the collection container (1) via another T-piece. A second tube (10) connects the second arm of each of the two T-pieces (2,3) with each other and with a pressure sensor (12). This is the case since in FIG. 1 the device is depicted in the state prior to use in a method for non-sterile waste removal. Therefore, the device is not yet connected to a non-sterile waste disposal site. A third tube (11), connects the third arm of each of the two T-pieces (2,3) via valves (6) and (8), respectively, with the aid of another T-piece with a sanitization source (4) here comprising 1 M NaOH. Moreover, the device comprises three pumps i.e. (13), which is used to mix the fluid in the collection container (1), as well as (14) and (15), which can alternatively draw fluid from the collection container into the first (9) and second (10) tubes or from the sanitization source (4) into the second (10) and third tubes (11). Prior to using the device the complete flow path is flushed with sanitization medium e.g. via decoupling pump (14) and using pump (15) for drawing 1 M NaOH via valves (5) and (8) into the collection container (1). While doing so valves (6) and (7) are closed. Alternatively the sanitization medium can also be drawn via valves (6) and (7), if valves (5) and (8) are closed and pump (14) is decoupled and pump (15) is used for drawing 1 M NaOH. Flushing and thus dearation, which is achieved via the flushing, of the flow path is completed, if a certain volume of sanitizing medium in flushed at a reasonably high flow rate into the collection container (1). Subsequently, the connection to the non-sterile waste disposal site is established e.g. via cutting out the pressure sensor (12). In this example pressure sensor (12) is used during flushing for safety reasons to detect high pressure.

Instead of cutting a disconnector could be used for establishing a connection to the waste disposal site after the flushing with sanitization medium.

In a setting where the device only comprises one distributor comprising at least three attachment points for tubes and only one second tube, a hydrophobic or hydrophilic bioburden reduction filter could be mounted at the end of the second tube, which will establish the connection with the non-sterile waste disposal site. During initial flushing of the device with sanitization medium the filter is also flushed with sanitization medium and removed after flushing with sanitization medium is completed.

The volume required to ensure decontamination of the flow paths of the device determines the volume which is used for the above described flushing of the device. In one example of this step the device is flushed with 20x the hold-up volume of the flow path.

In another example the device described above is a unit operation in the context of a larger production plant. In detail, the production plant comprises several flow paths. Some of these flow paths are pooled before they lead the collection container (1) but also a single flow path can lead directly to the collection container (1). The collection container (1) is connected to a scale to enable level monitoring and control. Draining liquid from collection container (1) to the drain is triggered by a certain upper filling level, and is stopped by reaching a certain lower filling level. The collection container comprises a vent filter to prevent the collection container (1) from bursting, if air is pumped into the collection container (1). Since air has a low density it cannot be measured by the scale.

### FIGURES

FIG. 1 shows schematically a process diagram of one embodiment of the device described herein, prior to use. In detail in this example the device comprises a GE Healthcare 20 L Hanging/Pillow Bag collection container (1), which is able to hold 20 liters. A first tube (9) - here a type C-Flex 374 and Pharmed BPT, both ¼" inner diameter - connects the first arm of each of the two T-pieces (2,3) via valves (5) and (7) - here type ACRO Versagrip 1450 valves -with the collection container (1). A second tube (10) connects the second arm of each of the two T-pieces (2, 3) with each other and with a pressure sensor (12) - here a type Pendotech single use pressure sensor. A third tube (11), made of C-Flex 374 and Pharmed BPT, connects the third arm of each of the two T-pieces (2,3) via valves (6) and (8), respectively, with a sanitization source (4) here a type 200 L Sartorius Flexel 3D bag comprising 1 M NaOH.
   Moreover, the device comprises three pumps here peristaltic dispenser pumps i.e. (13), which is used to mix the fluid in the collection container (1), as well as (14) and (15),
   Prior to using the device the complete device is flushed with sanitization medium.

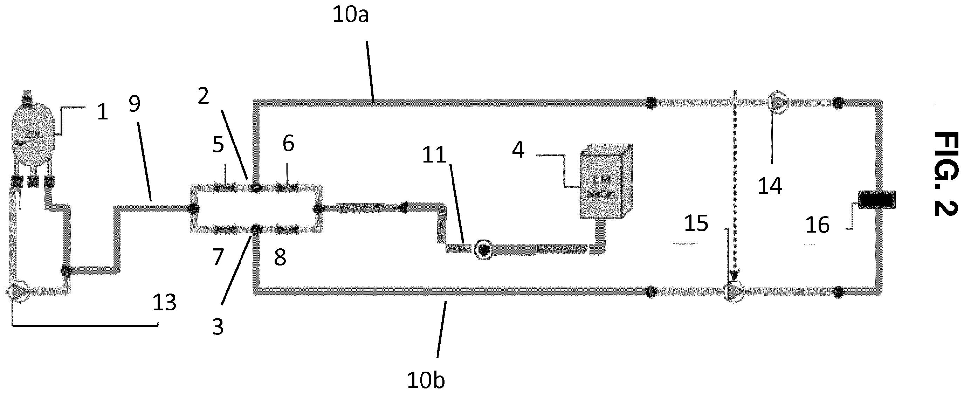

FIG. 2 shows a schematic drawing of the device of FIG. 1 in use. In detail, the pressure sensor (12) has been removed resulting in the second tubes (10a and 10b respectively) now connecting the distributors comprising at least three attachment points for tubes with the non-sterile waste disposal site (16), here a drain.

Thus, in a first state of the device fluid can be drawn by pump (14) from the collection container (1) via valve (5) into the non-sterile waste disposal site (16). In this setting valve (5) is opened and valves (7) and (6) are closed. Moreover, valve (8) is opened, because as pump (14) draws fluid from the collection container (1) into the non-sterile waste disposal site (16) pump (15) draws sanitization medium via valve (8) into the non-sterile waste disposal site (16) thereby decontaminating this flow path. After a predetermined volume of sanitization medium has been drawn from the sanitization source (4) - here 10x the filling volume of tubes (10b) and (11)- the process is switched with valves (6) and (7) being opened and valves (5) and (8) being closed.

## Claims

1. A device comprising at least one pathogen-reduced collection container, which is in fluid connection with at least one non-sterile waste disposal site via
- at least one distributor comprising at least three attachment points for tubes,
- at least three tubes, one of which connects the collection container with the first of said at least three attachment points for tubes of the at least one distributor,
- the second tube connects the second of said at least three attachment points for tubes of the at least one distributor with the non-sterile waste disposal site, and
- the third of said three tubes connects the third of said at least three attachment points for tubes of the at least one distributor with at least one sanitization source, comprising sanitization medium to decontaminate at least part of the second tube.

2. The device according to claim 1 further comprising at least one pump that pumps the fluid of the collection container to the non-sterile waste disposal site and the sanitization medium through at least part of the second tube, respectively.

3. The device according to claim 1 or claim 2, wherein the at least one distributor comprising at least three attachment points for tubes comprises a T-piece and/or an Y-piece and/or a three-way valve for distribution of the fluid stream.

4. The device according to anyone of the preceding claims, wherein the at least one distributor comprising at least three attachment points for tubes further comprises a component selected from the group comprising at least two valves, a combined dual valve and at least one pump, a T-piece, a Y-Piece, a multiple-way-valve or at least two pumps acting as valves.

5. The device according to anyone of the preceding claims, wherein an air gap is present between the at least one non-sterile waste disposal site and the second tube which connects the second of said at least three attachment points for tubes of the at least one distributor with the non-sterile waste disposal site.

6. The device according to anyone of the preceding claims, wherein the device is a unit operation.

7. The device according to anyone of the preceding claims, wherein the device comprises at least two distributors comprising at least three attachment points for tubes **characterized in that** said distributors comprise two valves each.

8. The device according to anyone of the preceding claims, wherein the device comprises three pumps and two distributors comprising at least three attachment points for tubes **characterized in that** said distributors comprisetwo valves each.

9. Production plant comprising one or more devices according to any of claims 1 to 8.

10. Method for non-sterile waste removal from a microbe-reduced or sterile process using the device according to anyone of claims 1 to 9.

11. Method for non-sterile waste removal from a microbe-reduced or sterile process according to claim 10, wherein prior to initiating non-sterile waste removal from a microbe-reduced or sterile process the device is flushed with sanitization medium.
